(19) **Europäisches Patentamt / European Patent Office / Office européen des brevets**

(11) **EP 2 035 568 B1**

(12) **FASCICULE DE BREVET EUROPEEN**

(45) Date de publication et mention
de la délivrance du brevet:
**16.12.2009 Bulletin 2009/51**

(51) Int Cl.:
***C12P 7/06*** *(2006.01)*     ***C12P 5/02*** *(2006.01)*
***C12F 3/10*** *(2006.01)*

(21) Numéro de dépôt: **07786997.2**

(22) Date de dépôt: **03.07.2007**

(86) Numéro de dépôt international:
**PCT/EP2007/056667**

(87) Numéro de publication internationale:
**WO 2008/003692 (10.01.2008 Gazette 2008/02)**

(54) **PROCEDE DE PRODUCTION DE BIOETHANOL ET DE COPRODUCTION D'ENERGIE A PARTIR D'UNE MATIERE PREMIERE VEGETALE AMYLACEE**

VERFAHREN ZUR PRODUKTION VON BIOETHANOL UND ZUR KOPRODUKTION VON ENERGIE AUS EINEM STÄRKEHALTIGEN PFLANZLICHEN AUSGANGSMATERIAL

METHOD FOR THE PRODUCTION OF BIOETHANOL AND FOR THE COPRODUCTION OF ENERGY FROM A STARCHY PLANT STARTING MATERIAL

(84) Etats contractants désignés:
**AT BE BG CH CY CZ DE DK EE ES FI FR GB GR HU IE IS IT LI LT LU LV MC MT NL PL PT RO SE SI SK TR**
Etats d'extension désignés:
**AL BA HR MK RS**

(30) Priorité: **03.07.2006 FR 0652767**

(43) Date de publication de la demande:
**18.03.2009 Bulletin 2009/12**

(73) Titulaire: **Mahler, John**
**Old Brompton Road**
**London**
**SW5 OEE (GB)**

(72) Inventeur: **Mahler, John**
**Old Brompton Road**
**London**
**SW5 OEE (GB)**

(74) Mandataire: **Kohn, Philippe et al**
**Cabinet Philippe Kohn**
**30, rue Hoche**
**93500 Pantin (FR)**

(56) Documents cités:
**WO-A-83/01627**     **WO-A-2004/113549**
**GB-A- 191 122 411**     **US-A- 4 337 123**

Printed by Jouve, 75001 PARIS (FR)

**Description**

DOMAINE TECHNIQUE DE L'INVENTION

La présente invention concerne un procédé de production de bioéthanol et de coproduction d'énergie à partir d'une matière première végétale.

L'invention a notamment pour but de produire, à échelle industrielle, du bioéthanol a partir de plantes amylacées avec une cogénération ou coproduction d'énergie utilisant la biomasse de la plante à l'exemple de la production de bioéthanol à partir de la canne à sucre qui utilise la bagasse de la plante.

Ce procédé peut être utilisé, non seulement pour de nouvelles distilleries de bioéthanol, mais aussi dans des distilleries existantes en adaptant les installations existantes.

Parmi les différents procédés de production de bioéthanol à partir d'une matière première végétale, on distingue trois groupes : a) les ressources saccharifères, telles que la betterave sucrière, les tiges sucrées telles que la canne à sucre ou le sorgho, les fruits ; b) les ressources amylacées telles que les grains de maïs ou de blé, et c) les ressources lignocellulosiques.

ARRIÉRE PLAN TECHNIQUE

En fonction de la matière première végétale de départ, le procédé de production de bioéthanol comporte généralement trois grands groupes principaux d'opération, c'est-à-dire consécutivement A) la préparation d'un moût, puis B) la fermentation du moût en vue de l'obtention d'un moût fermenté, puis D) la distillation du moût fermenté en vue de la production de bioéthanol.

A ces trois grands groupes d'opérations, on peut ajouter un quatrième groupe général E) d'opérations qui consistent dans divers traitements des coproduits résultant de chacun de ces trois groupes principaux d'opérations.

L'ensemble des opérations A) de préparation du moût vise à préparer une pâte ou un jus comportant de la matière première végétale apte à être fermentée, c'est à dire une solution aqueuse de sucres fermentescibles par des levures, et ceci en visant l'obtention d'une concentration la plus élevée possible de manière à réduire les capacités des équipements nécessaires à la préparation du moût et aux autres opérations ultérieures, tout en prenant en compte la limitation de la production éventuelle d'inhibiteurs de fermentation.

Dans le cas de production à partir de ressources saccharifères, l'étape spécifique de la préparation du moût consiste en l'extraction du saccharose, par exemple par pressage, ou lavage à l'eau chaude selon des techniques connues permettant l'obtention directe d'un jus très fermentescible.

Dans le cas de production à partir de ressources amylacées, il faut généralement d'abord transformer le grain en sucre soluble et fermentescible, par exemple, selon les techniques de transformation de l'amidon qui sont par exemple connues en amidonnerie, ou encore selon les techniques dites de "la voie acide".

La fermentation B) est fondée sur l'activité de micro-organismes dont le métabolisme fermentaire conduit à leur oxydation incomplète en éthanol et en $CO_2$.

Les performances des opérations de fermentation sont essentiellement fonction du micro-organisme utilisé (ou des micro-organismes utilisés), du milieu de culture sur lequel agit le micro-organisme et du procédé utilisé. Le rendement en alcool, ou éthanol, dépend de la maîtrise de ces différents paramètres.

Ce sont essentiellement les levures qui sont utilisées industriellement pour la production de bioéthanol.

La composition du milieu de culture, à partir du moût, vise essentiellement à fournir au micro-organisme utilisé les conditions optimales pour son métabolisme et la production qui lui est demandée.

Les technologies de fermentation utilisées sont diverses et connues, et les progrès dans le domaine de la fermentation visent essentiellement à en améliorer la rentabilité générale tant en ce qui concerne la productivité, que le taux de transformation en faisant par exemple appel à des levures, à des enzymes spécifiques, etc.

Pour obtenir un mélange fermenté (MF) à partir de la matière première végétale (MPV), les étapes ou opérations A) et B) peuvent être regroupées et/ou remplacées par d'autres méthodes de production d'un mélange fermenté (MF).

Les techniques de distillation D) utilisées sont elles aussi parfaitement connues, par exemple celles utilisées dans la distillation de solutions alcooliques, et elles ne diffèrent les unes des autres que par le schéma de distillation et l'optimisation des bilans énergétiques en corrélation avec les besoins énergétiques de chaque opération.

On rappellera néanmoins que le coût de la distillation est directement lié à la teneur en éthanol, à la qualité du produit distillé, aux consommations d'énergies et que des efforts constants sont donc nécessaires pour disposer d'un moût fermenté à forte teneur en éthanol.

Les différentes opérations E) de traitements des coproduits qui résultent des trois groupes principaux d'opérations décrits ci-dessus ont un impact important, tant en ce qui concerne l'économie des différents procédés de production de bioéthanol, que en ce qui concerne les aspects dits "environnementaux".

Quelle que soit la ressource en matière première végétale utilisée, tous les procédés aboutissent à l'obtention,

comme coproduits, de $CO^2$ et de la biomasse.

Dans le cas d'un procédé à partir de ressources saccharifères, par exemple à partir de la canne à sucre ou de la betterave sucrière, le glucose contenu dans la plante, qui est obtenu par broyage ou par pressage ou lavage à l'eau chaude, est directement fermentescible et les vinasses issues de la fermentation sont riches en matières organiques (+/-80%) et en matières minérales (+/-20%) qui posent des problèmes pour leur élimination.

Dans le cas de la canne à sucre, la bagasse, qui constitue la biomasse restant dans les moulins après extraction du jus sucré, peut être brûlée pour la coproduction d'énergie et sa combustion couvre les besoins en chaleur et en électricité des unités de production du bioéthanol, du fait du pouvoir calorifique de ce type de biomasse.

Dans le cas d'un procédé à partir de ressources amylacées, l'amidon contenu dans le grain doit d'abord être converti en sucre(s) fermentescibles, par exemple par la mise en oeuvre de la méthode enzymatique, de la méthode acide, ou de la méthode au malt.

Ainsi, la vinasse dite "brute" obtenue après distillation comporte essentiellement de l'eau et de la biomasse avec des levures produites au cours de la fermentation. La digestibilité permet d'en faire notamment un complément nutritionnel.

C'est ainsi que, après séparation de la fraction solide de la vinasse, par exemple par centrifugation puis par déshumidification et concentration de la fraction liquide de la vinasse, on obtient le produit dit "DDGS" (Distiller Dried Grain with Soluble) qui est utilisé notamment pour l'alimentation animale.

La vinasse peut aussi être utilisée pour la production d'agents fertilisants ou elle peut encore être transformée en énergie.

Des tentatives ont été menées pour brûler la vinasse après concentration, ou en l'envoyant dans des réacteurs pour produire du gaz méthane.

Toutefois, les problèmes techniques rencontrés -tels que par exemple le colmatage ou bouchage des tubes des chaudières - n'ont pas permis une mise en oeuvre rentable à l'échelle industrielle de tels types de traitements de la vinasse.

L'ensemble des procédés de production connus de bioéthanol à partir de plantes amylacées et sucrières présentent ainsi un bilan économique, et notamment énergétique, encore insuffisant ainsi qu'un bilan environnemental très négatif.

On a proposé dans le document US-A-4.337.123 de 1982 un procédé de production d'alcool carburant à partir de plantes fermentées "sans vinasse".

Ce procédé propose, après la fermentation et avant la distillation, de mettre en oeuvre un traitement au cours duquel plusieurs substances contenues dans le moût fermenté sont ôtées de façon que les moyens de distillation soient alimentés par un jus "purifié" afin que l'étape de distillation ne produise que de l'alcool et ne produise pas de vinasse.

Le procédé décrit dans ce document fait ainsi appel à un traitement par précipitation chimique, notamment par ajout d'un agent floculant, puis à une opération de décantation.

Un tel procédé est particulièrement complexe à mettre en oeuvre, coûteux, et il ne présente notamment aucun bilan énergétique favorable, tout en nécessitant d'utiliser des nouveaux produits supplémentaires pour l'obtention de la précipitation chimique.

Le bilan énergétique défavorable est notamment dû au fait que l'ensemble des produits "solides" séparés par décantation comporte une proportion de matière sèche très insuffisante pour que leur combustion ultérieur soit d'un rendement suffisant, c'est-à-dire que les opérations de séchage préalable à cette combustion nécessitent l'apport d'une quantité d'énergie fossile externe trop importante. En d'autres termes, l'humidité de l'ensemble des produits "solides" séparés par décantation (ou phase solide) est trop élevée pour que le procédé présente un bilan énergétique satisfaisant.

On a proposé dans le document EP-A2-0.048.061, de 1981, un procédé et un appareil de traitement de la vinasse dans le cadre d'un procédé général de production d'alcool à partir de la canne à sucre, en vue d'optimiser le bilan énergétique global du procédé de production de l'alcool.

Ce procédé propose de concentrer des matières solides et solubles contenues dans la vinasse, puis de les brûler pour obtenir de la vapeur réutilisée sous différentes formes, notamment dans le procédé de production d'alcool.

La combustion de la vinasse concentrée est très difficile et doit être réalisée dans des chaudières très complexes et coûteuses, similaires aux chaudières que l'on utilise dans l'industrie de la cellulose pour brûler la liqueur noire ("black liquor") concentrée.

On a proposé dans le document WO-A1-2004/113549, des procédés de production d'éthanol et de méthane à partir de la biomasse dont les performances sont améliorées par contrôle et modifications des paramètres de la biomasse utilisée. Un des procédés consiste en variante, préalablement à la fermentation ou à là distillation, à séparer de la biomasse les protéines qui y sont présentes, ainsi que le son qui pourrait y être présent.

RÉSUMÉ DE L'INVENTION

La présente invention vise à proposer un nouveau procédé de production de bioéthanol et de coproduction d'énergie, à partir d'une matière première végétale amylacée et se caractérisant essentiellement en ce qu'il comporte une étape, intervenant avant la distillation, de filtration, de lavage et de pressage permettant de séparer la phase liquide de la phase solide du moût fermenté.

L'invention propose ainsi un procédé de production de bioéthanol et de coproduction d'énergie, à partir d'une matière première végétale MPV amylacée, procédé du type comportant au moins les étapes suivantes successives consistant à :

- A) -B) obtenir à partir de toute la matière première végétale (MPV) un mélange fermenté (MF) ;
- C1) séparer, par filtration et pressage, la phase liquide (PL) et la phase solide (PS) du mélange fermenté (MF), de telle manière que la proportion en poids de la matière sèche de ladite phase solide (PS) est comprise entre environ 40% et environ 45% ;
- D) distiller au moins en partie ladite phase liquide (PL) dudit mélange fermenté (MF) pour obtenir de l'éthanol et de la vinasse légère (VL) ;
- E1) produire, en utilisant toute ladite vinasse légère (VL), du gaz méthane (F1) constituant un premier combustible pour la coproduction d'énergie;

et en ce que, préalablement à ladite étape C1) de séparation, le procédé comporte :

- une étape au cours de laquelle le pH dudit mélange fermenté (MF) est augmenté pour être porté à une valeur comprise entre environ 5,5 et environ 6,5 ;

et une étape au cours de laquelle on ajoute audit mélange fermenté (MF) un adjuvant de filtration (ADJ).

Ladite étape E1) de production d'au moins un premier combustible consiste à produire du gaz méthane à partir de toute la vinasse légère VL, et éventuellement à partir des flegmasses provenant de la rectification et la déshydratation de l'éthanol.

Grâce à l'invention, il est possible d'obtenir une biomasse qui est brûlée pour produire de l'énergie, et dont le pouvoir calorifique est analogue à celui de la bagasse et, comme dans le cas de l'alcool produit à partir de la canne à sucre, quasiment sans nécessiter de faire appel à une énergie fossile externe.

De plus, les qualités de la vinasse légère obtenue après distillation sont telles qu'elles permettent de produire du méthane dans des conditions optimales de rendement.

Après méthanisation, la phase liquide ainsi obtenue peut subir un traitement complémentaire aboutissant à la production d'eau réutilisable dans la mise en oeuvre du procédé selon l'invention ou rejetée dans le milieu naturel. La qualité de ces eaux répondent aux exigences et normes environnementales les plus sévères. La méthanisation produit aussi des boues en faible quantité qui après séchage peuvent constituer, par exemple, des produits d'amendements des sols.

Des traitements supplémentaires permettent la réutilisation des eaux dans le cadre de la mise en oeuvre du procédé selon l'invention, grâce à la très faible quantité de charges polluantes en sortie de méthanisation.

Selon une autre caractéristique du procédé selon l'invention, il comporte une étape E2) de production d'au moins un deuxième combustible qui consiste à déshumidifier ladite phase solide PS du mélange fermenté MF pour produire un bloc de matériau dont la proportion en poids de matière sèche est supérieure à 50%, ledit bloc étant apte à être brûlé, en totalité ou en partie, dans une chaudière, et/ou ledit bloc étant apte à être utilisé, en totalité ou en partie, pour la production d'un produit (DDGS) utilisé notamment pour l'alimentation animale.

Ladite phase solide PS du mélange fermenté MF est par exemple déshumidifiée par séchage.

Toutefois, cette opération de séchage ne nécessite que très peu d'énergie qui peut par exemple être constituée par l'énergie thermique contenue dans les fumées FUM de la chaudière. Le séchage ne nécessite donc pas d'énergie fossile externe, ni de vapeur produite dans le cadre du procédé selon l'invention, et le pouvoir calorifique du bloc "séché" est ainsi encore augmenté de manière très économique du point de vue du bilan énergétique global du procédé.

Les gaz (G) émis par la phase solide lors du séchage sont traités pour extraire, en totalité ou en partie, l'éthanol que ces gaz contiennent, au moyen de procédés incluant, mais non limités à, le lavage à l'eau des gaz, le passage des gaz sur un charbon actif, etc.

On augmente encore ainsi le rendement du procédé en matière de production d'éthanol.

Selon unes autre caractéristique du procédé selon l'invention, ledit premier combustible que constitue le méthane, en totalité ou en partie, et ledit deuxième combustible (le bloc de matériau obtenu à partir de la phase solide), en totalité ou en partie, sont brûlés dans une même chaudière. Cela permet une meilleure combustion du bloc, ou "cake", tout en utilisant une chambre de combustion de plus petites dimensions.

Selon un autre aspect de l'invention, la très grande efficacité de l'étape C1) de séparation de la phase liquide PL et de la phase solide PS du mélange fermenté MF, permettant que la proportion en poids de la matière sèche de ladite phase solide (PS) soit comprise entre environ 40% et environ 45%, est avantageusement obtenue en ce que ladite étape de séparation C1) est réalisée au moyen d'un filtre-presse adapté à cette usage.

Toujours pour améliorer les performances du processus de séparation des phases liquide et solide, c'est à dire pour augmenter l'aptitude du mélange fermenté à être filtré :

- préalablement à ladite étape C1) de séparation, le procédé comporte une étape au cours de laquelle la température du mélange fermenté est portée à une température T de séparation, comprise entre environ 55°C et environ 65°C ;
- le pH du mélange fermenté (MF) est par exemple augmenté par ajout d'au moins un composant alcalin ;

Le procédé comporte une étape intermédiaire C2) de lavage de ladite phase solide séparée du mélange fermenté, de manière à récupérer la plus grande quantité possible de l'éthanol résiduel contenu dans la phase solide.

Cette étape C2) de lavage de la phase solide séparée PS du mélange fermenté est avantageusement réalisée par injection d'eau de lavage dans le filtre-presse de telle manière qu'au moins une partie du liquide de lavage LL très riche en éthanol est ajoutée automatiquement à la phase liquide du mélange fermenté à distiller.

Le procédé selon l'invention permet, industriellement, de produire simultanément du bioéthanol et de l'énergie, notamment du fait de la maîtrise de la proportion de matière sèche de la phase solide et du fait de la qualité (quasi absence de matières solides en suspension) de la phase liquide avant distillation.

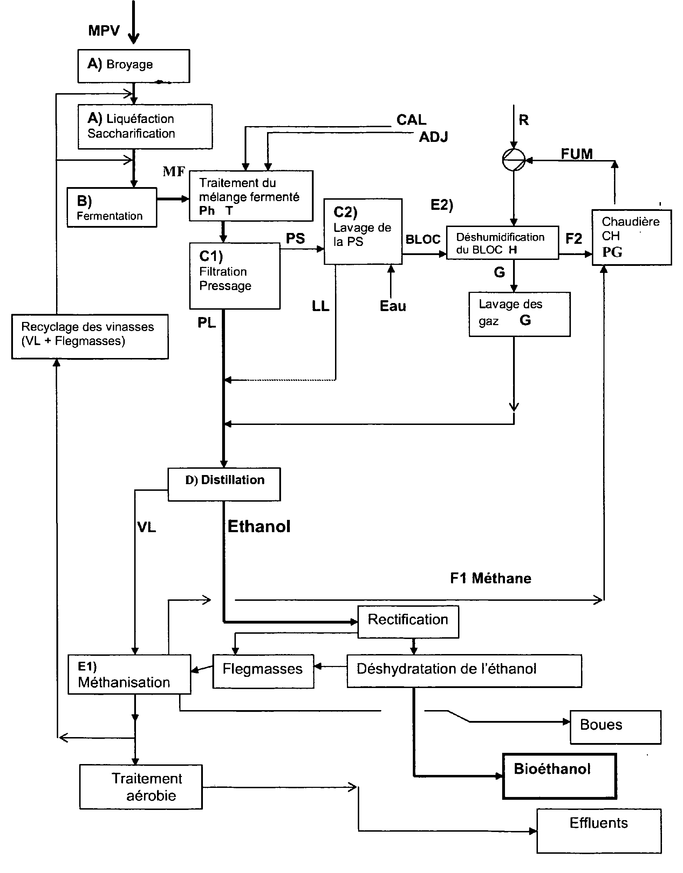

## BRÈVE DESCRIPTION DE LA FIGURE

D'autres caractéristiques et avantages de l'invention apparaîtront à la lecture de la description qui va suivre, donnée à titre d'exemple non limitatif, pour la compréhension de laquelle on se reportera au dessin annexé dans lequel la figure unique est un schéma illustrant un exemple d'un procédé selon l'invention.

## DESCRIPTION DÉTAILLÉE DES FIGURES

On décrira maintenant, en référence à la figure unique, un exemple de mise en oeuvre du principe de séparation/ filtration selon l'invention des phases liquide et solide qui est appliqué ici au moût fermenté avant distillation.

La matière première végétale MPV amylacée subit par exemple une première étape A de préparation d'un moût.

Il s'agit par exemple, lorsque la matière première végétale MPV est une céréale, de sous-étapes de broyage des céréales, puis de saccharification et liquéfaction du mélange broyé.

La matière première végétale MPV peut être constituée directement de grains tels que du maïs ou du blé, le broyage aboutissant alors à la préparation d'une farine qui est elle-même préparée en vue d'obtention du moût.

Le moût est ainsi une pâte élaborée à partir de la matière première végétale MPV qui est apte à être fermentée.

Le procédé comporte ensuite une étape B de fermentation du moût en vue d'obtenir un mélange fermenté MF apte à être distillé, aussi appelé le moût fermenté MF.

De manière connue, le dioxyde de carbone $CO_2$ est un coproduit d'une telle étape B de fermentation.

Le procédé comporte ensuite l'étape D de distillation permettant d'obtenir le bioéthanol, c'est-à-dire le produit principal du procédé comportant les étapes successives A, B et D, ainsi qu'un coproduit appelé vinasse qui est un mélange, notamment riche en eau.

A l'issue de l'étape B de fermentation, le moût fermenté ME subit immédiatement, c'est-à-dire avant la distillation D, et au cours d'une opération intermédiaire C1, une opération de séparation physique de la phase liquide PL et de la phase solide PS du moût fermenté MF.

La phase liquide PL du moût fermenté MF est envoyée à la distillation, c'est-à-dire qu'elle subit l'étape D de distillation aboutissant à la production de bioéthanol et à la production d'un coproduit liquide ici appelé la vinasse légère VL.

Le fait que, conformément aux enseignements de l'invention, l'opération de distillation soit appliquée à la seule phase liquide PL du moût fermenté MF permet notamment que l'on utilise des équipements de dimensions et de capacités réduites par rapport à des opérations classiques de distillation d'un produit à deux phases, liquide et solide, mélangées.

La séparation de la phase liquide PL du moût fermenté MF est obtenue mécaniquement par filtration et pressage, de préférence au moyen d'un filtre-presse, et/ou en variante d'un filtre et d'une presse fonctionnant en continu ou en discontinu.

Ces premières opérations physiques aboutissant à la séparation de la phase liquide PL et de la phase solide PS du moût fermenté sont désignées à la figure par l'étape C1.

La qualité de la séparation réalisée selon l'invention dépend de la capacité ou aptitude du mélange fermenté MF à être filtré.

Cette aptitude peut par exemple être exprimée sous la forme du paramètre dit "CST" qui est mesuré selon des méthodes normalisées bien connues de l'homme de l'art.

Dans le cadre de la présente invention, il a été découvert que la maîtrise et/ou la modification de certains paramètres du mélange fermenté obtenu à partir de la matière première amylacée augmente de manière importante cette aptitude à être filtré, et donc la proportion en poids de la matière sèche obtenue.

Le premier de ces paramètres est la température T, dite ici température de filtration, du mélange lorsqu'il est introduit dans les moyens de séparation utilisés, et par exemple dans un filtre-presse.

Ainsi, préalablement à l'étape de séparation, le procédé comporte une étape au cours de laquelle la température du mélange fermenté MF est portée, ou maintenue, à une température T de séparation qui est comprise entre environ 55°C et environ 65°C. Cette maîtrise de la température T de séparation peut résulter directement des étapes préalables de traitement de la matière première en vue de l'obtention du mélange fermenté, et peut par exemple être obtenue sans consommation d'énergie supplémentaire, le mélange fermenté devant de toutes manières être porté à 65°C avant distillation.

Le second de ces paramètres est le pH du mélange lorsqu'il est introduit dans les moyens de séparation utilisés.

Ainsi, préalablement à l'étape de séparation, le procédé comporte une étape au cours de laquelle le pH du mélange fermenté MF est augmenté pour être porté à une valeur comprise entre environ 5,5 et environ 6,5. Par exemple, le pH du mélange fermenté MF est augmenté par ajout d'au moins un composant alcalin incluant, mais non limité au, le carbonate de calcium CaCo3 ou l'hydroxyde de calcium Ca(OH)2.

On constate de plus que ces deux paramètres (Température T et pH) sont liés quant à l'aptitude du mélange fermenté à être séparé, c'est-à-dire que l'on peut établir une série de courbes indiquant la valeur du CST en fonction de la température T, et pour une valeur donnée du pH (ou inversement).

De plus les valeurs de ces paramètres dépendent de la matière première amylacée utilisée.

Il est encore possible d'améliorer l'aptitude du mélange à être filtré en faisant appel à un adjuvant ADJ de filtration, par exemple à base de polymères.

Les sous-produits solides résultant de la séparation physique en C1 peuvent subir, comme illustré ici, une sous étape C2 de lavage des produits solides séparés.

Le lavage est effectué par exemple par injection d'Eau de lavage dans le filtre-presse, avec au minimum la même température que celle du mélange fermenté MF. Après lavage, l'Eau de lavage est appelée le liquide de lavage LL et ce liquide de lavage est réutilisé de la manière suivante.

Le liquide de lavage LL à forte teneur en éthanol est réutilisé en tout ou partie en étant mélangé à la phase liquide PL du moût fermenté MF avant la distillation D.

Dans le cas de l'utilisation du filtre-presse, ce "mélange" est automatique à la "sortie" du filtre-presse.

On récupère ainsi une partie de l'éthanol contenu dans la phase solide PS. La récupération de cet éthanol à un stade ultérieur serait plus complexe et coûteuse.

Conformément au procédé selon l'invention de production de bioéthanol et de coproduction d'énergie, la vinasse légère VL subit ensuite une étape E1 de production d'un premier combustible F1 qui est ici du méthane.

Cette étape E1, dite de méthanisation, est ainsi appliquée à de la vinasse légère VL dont les qualités sont optimales à cet effet, notamment en ce que la vinasse ne contient pratiquement aucun composant solide en suspension.

La production du gaz méthane ou biogaz est par exemple obtenue par traitement par voie anaérobie. Par acidogenèse et méthanogenèse, on obtient du méthane qui constitue le premier combustible F1 obtenu selon le procédé de l'invention qui peut ensuite être utilisé, lors d'une étape PG, pour coproduire de l'énergie.

La production de gaz méthane par méthanisation, à partir de la vinasse liquide - dite vinasse légère VL - issue de la distillation ainsi que à partir des "flegmasses" FG résultant des étapes connues de rectification et de déshydratation de l'éthanol après l'étape de distillation.

La vinasse légère VL a une faible teneur en azote parce que l'azote, présente dans la matière première végétale amylacée et dans les levures utilisées pour la fermentation, a été éliminée en grande partie grâce à l'opération de séparation des phases liquide et solide avant la distillation. Cette méthanisation est particulièrement avantageuse et efficace dans la mesure où la phase liquide a une faible teneur en azote, l'azote étant un inhibiteur de la méthanisation.

Au cours de l'étape PG, des équipements incluant, mais non limités à : un générateur ; une chaudière ; une turbine à gaz ; un moteur, alimentés en méthane peuvent produire de l'énergie incluant, mais non limitée à : l'électricité ; la vapeur d'eau ; l'eau chaude, etc.

Dans l'exemple illustré à la figure unique, le méthane F1 est brûlé dans une chaudière qui est par exemple une chaudière pour la production de vapeur d'eau. La chaudière produit aussi des fumées résiduelles FUM.

On dispose ainsi d'un cycle très efficace de coproduction d'énergie à partir du combustible issu de la vinasse légère VL.

Les effluents liquides produits lors de l'étape E1 de la gazéification (méthanisation) peuvent être traités, au cours d'une ou plusieurs étapes de traitement, par voie aérobie complémentaire, entre autres afin d'obtenir un effluent liquide épuré et/ou des eaux réutilisables dans le procédé selon l'invention.

Dans le cadre de la coproduction, ou cogénération, d'énergie suivant le procédé selon l'invention, la phase solide PS du moût fermenté MF, c'est-à-dire les matières résiduelles provenant de la fermentation B, est elle aussi aisément transformée en énergie.

Du fait de la technique de séparation par filtration et pressage, notamment dans un filtre-presse, la proportion en poids de la matière sèche de la phase solide PS obtenue est supérieure à 40% en poids, et est par exemple comprise entre environ 40% et environ 45%.

L'étape E2, de production du deuxième combustible est une étape de déshumidification, par exemple par séchage et/ou par tout autre procédé physique adapté, qui consiste à déshumidifier la phase solide PS du moût fermenté MF pour produire un bloc ou tourteau séché F2, aussi appelé "cake", qui est alors un élément combustible apte à être aisément brûlé.

En effet, l'étape de déshumidification permet de disposer d'un bloc combustible dont la teneur en matière sèche est alors supérieure à 50%, c'est-à-dire un taux permettant une bonne combustion.

Le bloc constitue ainsi, au sens de l'invention, le deuxième produit combustible F2 pour la coproduction d'une seconde énergie au cours d'une deuxième étape de coproduction d'énergie.

Ce combustible F2 peut ainsi par exemple être brûlé dans une chaudière qui produit de l'énergie incluant, mais non limitée à : l'électricité ; la vapeur d'eau ; l'eau chaude, etc.

Ce combustible F2 est ici de préférence brûlé dans une chaudière qui est ici la même chaudière CH que celle dans laquelle on brûle le méthane F1.

Les moyens utilisés pour les coproductions PG d'énergie rejettent aussi des fumées FUM et/ou des gaz qui peuvent être récupérés au cours d'une étape R et qui peuvent notamment être utilisés comme source d'énergie lors de l'étape E2 de déshumidification de la phase solide.

La chaleur contenue dans ces fumées est récupérée au moyen d'un échangeur. Cette énergie de séchage est ainsi économique parce qu'elle est récupérée sans qu'il soit nécessaire de faire appel à de la vapeur produite par les chaudières dans le cadre du procédé, ni à de l'énergie fossile externe.

On notera aussi que cette étape aboutit à la production de cendres.

Grâce aux deux étapes E1 et E2 de production des deux combustibles F1 et F2 qui sont ensuite transformés en énergie, le procédé selon l'invention est un procédé de production de bioéthanol et de coproduction d'énergie PG, car non seulement la production de bioéthanol peut être "autosuffisante" en énergie, mais le procédé aboutit à la coproduction d'un excédent d'énergie qui peut être commercialisée sous les formes incluant, mais pas limitées à, la vapeur d'eau, l'eau chaude, l'électricité, etc.

La combustion de la phase solide PS à haute teneur en matière sèche peut être effectuée avec facilité dans une chaudière pour biomasse, si on compare cette combustion à toutes les tentatives précédentes de combustion de vinasses concentrées, sans séparation préalable des phases solide et liquide.

Les résidus de la combustion des deux combustibles, ou des combustions si elles sont effectuées séparément, peuvent être commercialisés après séchage, par exemple sous la forme de produits d'amendement des sols.

En fonction de différents paramètres, et notamment de la matière première végétale MPV utilisée, on peut utiliser la phase liquide PL en totalité ou en partie en vue de la production du méthane F1.

De même, on peut utiliser la phase solide PS en totalité ou en partie en vue de la production du combustible solide ou bloc F2, et/ou l'utiliser en totalité ou en partie pour la production de DDG (Distiller Dried Grain) qui est utilisé notamment pour l'alimentation animale.

Ce DDG est d'une qualité bien supérieure à celle actuellement disponible, notamment parce que la teneur en éthanol résiduel est très faible.

Cette teneur résiduelle très faible résulte d'abord de la technique de séparation utilisée.

La teneur en éthanol du bloc F2 est encore réduite grâce à une étape C2) de lavage de la phase solide PS séparée du mélange fermenté MF.

Si le lavage est effectué à l'extérieur des moyens de filtration et de pressage, le liquide de lavage peut être réutilisé en tout ou partie en étant mélangé au moût en amont de l'étape B de fermentation. Il est ainsi possible de mélanger le liquide au moût avant l'étape de fermentation et/ou de l'utiliser pour l'étape de préparation. On réalise ainsi de plus une économie d'une partie de l'eau utilisée pour la préparation et/ou la fermentation.

Une séparation du liquide de lavage, après lavage, en deux voies distinctes, peut être effectuée en fonction de la teneur en éthanol du liquide de lavage.

Avantageusement, lorsque la séparation de PL et de PS est effectuée au moyen d'un filtre presse, la sous étape C2) de lavage de la phase solide séparée PS du mélange fermenté MF est réalisée par injection d'eau de lavage dans le filtre-presse de telle manière qu'au moins une partie du liquide de lavage à teneur en éthanol est alors ajoutée "automatiquement" à la phase liquide PL du mélange fermenté à distiller.

La teneur en éthanol du bloc combustible F2 est encore réduite au cours de l'étape de déshumidification par séchage qui provoque une évaporation sous forme de gaz G de la matière liquide qu'il contient, et notamment de l'éthanol qui est alors sous forme de vapeurs d'alcool.

Cet éthanol "vaporisé" peut lui aussi être récupéré par exemple par une étape de lavage des gaz G, par exemple au moyen d'eau.

## EXEMPLE D'UN BILAN DE PRODUCTION D'ÉTHANOL ET DE COPRODUCTION D'ÉNERGIE SELON L'INVENTION

On présentera maintenant à titre non limitatif, un exemple du bilan de production de bioéthanol et de coproduction d'énergie(s) obtenues après un essai pilote.

La matière première végétale envisagée est du blé qui contient par hypothèse 12.8% en poids d'eau et 87.2% en poids de matière sèche parmi laquelle 59% en poids de la MPV est de l'amidon.

Avec un tel blé, pour obtenir 100 litres, soit un hectolitre, de bioéthanol, il faut 272.9 kg de blé, qui contiennent 34.9 kg d'eau, 161.0 kg d'amidon et 77.0 kg d'autres matières sèches.

Après saccharification/liquéfaction, fermentation et distillation on obtient 100 litres de bioéthanol et 92.0 kg de matières sèches.

A partir des 92.0 kg de matières sèches, après séparation des phases solide et liquide, on dispose de 60,37 kg de matière comme combustible à brûler dans une chaudière, et la phase liquide est distillée et on dispose de 31,63 kg de matière pour la méthanisation pour la production de gaz méthane pouvant aussi être utilisé dans une chaudière.

E2) Pour les 60,37 kg de phase solide séparée ou "cake" :

« Pouvoir Calorifique Inférieur » (PCI) pour 50% de matière sèche du cake : 2.150 kcal/kg

Energie récupérée :

120.74 kg x 2.150 kcal/kg :1000 = 259.59 thermies
259.59 thermies x 1,163 = 301.90 kWh
301.90 kWh x 3,6 = 1.086,84 MJ

E1) Pour les 31,63 kg de matières organiques et minérales solubilisées dans la vinasse légère :

Hypothèse: Demande Chimique en Oxygène (02) utilisable = 92 g/l de vinasse.

Quantité de moût fermenté a 12,54 % volume :

100 litres : 12,54 x 100 = 797,48 litres, on considérera 797,50 litres.

Considérant un recyclage de la vinasse de 12,8 % :

797,50 litres - (797,5 litres x 12,8 :100) = 695,42 litres, on considérera 695,50 litres

Production d'alcool (bioéthanol) a 92,5 % volume nécessaire pour produire 100 litres d'alcool pur :

100 litres : 92,5 x 100 = 108,1 litres, on considèrera 108 litres.

Quantité de vinasse légère produite pour 100 litres d'alcool pur.

695,5 litres - 108 litres = 587,50 litres de vinasse légère pour 100 litres d'alcool pur.

Energie récupérée avec le méthane pour 100 litres d'alcool pur :

92 g/l x 587,5 litres : 1.000 = 54,05 kg.
54,05 kg x 0,446 x 6.450 kcal/kg = 155,486 kcal

(dans laquelle 0,446 est le coefficient de rendement de la méthanisation)

155,486 kcal :1.000 x 1,163 = 180,83 kWh
180,83 kWh x 3,6 = 651 MJ

Energie totale E1 + E2 :

301,90 kWh + 180,83 kWh = 482,73 kWh

Besoins en énergie pour la production de 100 litres d'alcool pur :

Energie électrique : 25 kWh
Energie thermique : 166,32 kWh

Considérant le rendement de la chaudière à vapeur de 92% :

$$482{,}73 \text{ kWh} \times 92 : 100 = 444{,}11 \text{ kWh}$$

Considérant qu'une turbine à contre-pression a un rendement de 83 %, soit 20 % en énergie électrique et 63 % en énergie thermique :

La capacité de la turbine devra être de :

$$166{,}32 \text{ kWh} \times 63 : 100 = 264 \text{ kWh}$$

La production d'énergie électrique de la turbine est de :

$$264 \text{ kWh} \times 20 : 100 = 52{,}80 \text{ kWh}$$

Solde disponible d'énergie électrique commercialisable :

$$52{,}80 \text{ kWh} - 25 \text{ kWh} = 27{,}80 \text{ kWh}$$

Energie encore disponible :

$$444{,}11 \text{ kWh} - 264 \text{ kWh} = 180{,}11 \text{ kWh}$$

Considérant qu'une turbine à condensation contre-pression a un rendement de 32 % en énergie électrique :

$$180{,}11 \text{ kWh} \times 32 : 100 = 57{,}64 \text{ kWh}$$

Energie électrique totale disponible pour commercialisation :

27,80 kWh + 57,64 kWh = 85,44 kWh pour 100 litres d'alcool pur produits.
Observation : le rendement énergétique du gaz peut être considérablement augmente avec l'utilisation d'une turbine a gaz a cycles combines.

A titre comparatif :

Une tonne de canne a sucre permet la production de 90 litres d'alcool pur.
Une tonne de canne a sucre permet de produire un excèdent d'énergie électrique commercialisable de 75 kWh par tonne de canne (fonte Celso Procknor, Magasine STAB/Brésil édition janvier 2007).
Excèdent d'énergie électrique commercialisable pour 100 litres d'alcool pur :

$$75 \text{ kWh} : 90 \text{ litres} \times 100 \text{ litres} = 83{,}33 \text{ kWh}$$

En conclusion, la présente invention permet de produire avec la biomasse des plantes amylacées autant d'énergie que celle fournie par la bagasse de qu'avec la canne a sucre.

Balance énergétique pour 1 tonne de céréales dans le cas de l'essai pilote :

1 tonne de céréales permet de produire 400 litres d'alcool pur.

| | |
|---|---|
| Culture des céréales : | -1.367 MJ |
| Stockage des céréales : | - 150 MJ |
| Production d'alcool : | -2.500 MJ |
| Production de biogaz : | - 450 MJ |
| Energie éthanol : | +8.480 MJ |
| Energie du "cake" : | +4.348 MJ |
| Energie méthanisation : | +2.604 MJ |
| Gain d'énergie : | +10.965 MJ |

Rapport Energie produite/Energie utilisée :

$$15.432 \text{ MJ} : 4.467 \text{ MJ} = 3,45$$

Balance énergétique pour 1 tonne de céréales dans le cas de production de DDGS :

| | |
|---|---|
| Culture céréales : | -1.367 MJ |
| Stockage des céréales : | - 150 MJ |
| Production d'alcool : | -2.500 MJ |
| Séchage DDGS : | -2.400 MJ |
| Energie éthanol : | +8.480 MJ |
| DDGS : | +5.096 MJ |
| Gain d'énergie : | +2.063 MJ |

Rapport Energie produite/Energie utilisée :

$$8.480 \text{ MJ} : 6.417 \text{ MJ} = 1.32$$

En conclusion, le gain d'énergie obtenu par le procédé de production de bioéthanol et de coproduction d'énergie selon l'invention est en contradiction avec les critiques faites sur l'alcool obtenu à base de céréales par certains auteurs de l'art antérieur. L'énergie produite est nettement supérieure à l'énergie utilisée alors qu'elle est considérée nulle par ces mêmes auteurs.

Bilan des Eaux

Eau de refroidissement :

Hypothèse : tour de réfrigération d'eau
Besoins en eau pour 100 litres d'alcool pur produits : 0,8 m$^3$
Pertes : 6 %, soit 0,048 m$^3$, on considèrera 0,05 m$^3$/hl
Eau fraîche pour le procédé de fabrication : 0,4-0,5 m$^3$/hl
Eau des chaudières en considérant la récupération des condensats et un chauffage se faisant par échangeur thermique : 0,05 m$^3$/hl
Besoin total d'eau : 0,5 - 0,6 m$^3$/hl alcool pur produit

Cette quantité d'eau peut être réduite à environ 0,15 m$^3$/hl avec un traitement des effluents liquides provenant de la méthanisation.

**Revendications**

1. Procédé de production de bioéthanol et de coproduction d'énergie, à partir d'une matière première végétale (MPV) amylacée,
   **caractérisé en ce qu'**il comporte au moins les étapes suivantes successives consistant à :

   - A) -B) obtenir à partir de toute la matière première végétale (MPV) un mélange fermenté (MF) ;
   - C1) séparer, par filtration et pressage, la phase liquide (PL) et la phase solide (PS) du mélange fermenté (MF), de telle manière que la proportion en poids de la matière sèche de ladite phase solide (PS) est comprise entre environ 40% et environ 45% ;
   - D) distiller au moins en partie ladite phase liquide (PL) dudit mélange fermenté (MF) pour obtenir de l'éthanol et de la vinasse légère (VL) ;
   - E1) produire, en utilisant toute ladite vinasse légère (VL), du gaz méthane (F1) constituant un premier combustible pour la coproduction d'énergie;

   et **en ce que**, préalablement à ladite étape C1) de séparation, le procédé comporte :

   • une étape au cours de laquelle le pH dudit mélange fermenté (MF) est augmenté pour être porté à une valeur comprise entre environ 5,5 et environ 6,5 ;
   • et une étape au cours de laquelle on ajoute audit mélange fermenté (MF) un adjuvant de filtration (ADJ).

2. Procédé selon la revendication 1, **caractérisé en ce que** ladite étape de séparation C1) est réalisée au moyen d'un filtre-presse.

3. Procédé selon l'une quelconque des revendications précédentes, **caractérisé en ce que**, préalablement à ladite étape C1) de séparation, le procédé comporte une étape au cours de laquelle la température dudit mélange fermenté (MF) est portée à une température (T) de séparation, comprise entre environ 55°C et environ 65°C.

4. Procédé selon la revendication 1, **caractérisé en ce que** le pH dudit mélange fermenté (MF) est augmenté par ajout d'au moins un composant alcalin (CAL).

5. Procédé selon l'une des revendications 1 ou 2, **caractérisé en ce qu'**il comporte une sous-étape C2) de lavage de ladite phase solide (PS) séparée dudit mélange fermenté (MF).

6. Procédé selon la revendication précédente prise en combinaison avec la revendication 2, **caractérisé en ce que** ladite étape C2) de lavage de la phase solide séparée (PS) du mélange fermenté (MF) est réalisée par injection d'eau de lavage dans le filtre-presse de telle manière qu'au moins une partie du liquide de lavage (LL) à teneur en éthanol est ajoutée automatiquement à ladite phase liquide (PL) du mélange fermenté à distiller.

7. Procédé selon l'une quelconque des revendications précédentes, **caractérisé en ce qu'**il comporte une étape E2) de production d'au moins un deuxième combustible qui consiste à déshumidifier ladite phase solide (PS) dudit mélange fermenté (MF) pour produire un bloc (F2) de matériau dont la proportion en poids de matière sèche est supérieure à 50%, et **en ce que** ledit bloc (F2) est apte à être brûlé, en totalité ou en partie, dans une chaudière (CH), et/ou **en ce que** ledit bloc (F2) est apte à être utilisé, en totalité ou en partie, pour la production d'un produit (DDG) utilisé notamment pour l'alimentation animale.

8. Procédé selon la revendication précédente, **caractérisé en ce que** ladite phase solide (PS) dudit mélange fermenté (MF) est déshumidifiée par séchage (H), et **en ce que** les gaz (G) émis lors du séchage sont traités pour extraire, en totalité ou en partie, l'éthanol que ces gaz contiennent, notamment par lavage des gaz à l'eau.

9. Procédé selon les revendications 7 et 8, **caractérisé en ce que** ledit méthane (F1), en totalité ou en partie, et ledit deuxième combustible (F2), en totalité ou en partie, sont brûlés dans une même chaudière.

10. Procédé selon l'une quelconque des revendications précédentes, **caractérisé en ce que** ledit mélange fermenté (MF) est obtenu par les étapes successives consistant à :

- A) préparer une pâte comportant la matière première végétale (MPV) apte à être fermentée ;
- B) provoquer la fermentation de ladite pâte en vue d'obtenir ledit mélange fermenté (MF).

**Claims**

1. A method for the production of bioethanol and for the coproduction of energy from a starchy plant starting material (MPV),
   **characterized in that** it comprises at least the following successive steps consisting in:

   - A) -B) obtaining, from all of the plant starting material (MPV), a fermented mixture (MF);
   - C1) separating, by filtration and pressing, the liquid phase (PL) and the solid phase (PS) from the fermented mixture (MF), in such a way that the proportion by weight of the solids of said solid phase (PS) is between approximately 40% and approximately 45%;
   - D) distilling, at least in part, said liquid phase (PL) of said fermented mixture (MF) so as to obtain ethanol and light vinasse (VL);
   - E1) producing, using all of said light vinasse (VL), methane gas (F1) constituting a first fuel for the coproduction of energy;

   and **in that**, prior to said separation step C1), the method comprises:

   • a step during which the pH of said fermented mixture (MF) is increased so as to be brought to a value of between approximately 5.5 and approximately 6.5;
   • and a step during which a filtration adjuvant (ADJ) is added to said fermented mixture (MF).

2. The method as claimed in claim 1, **characterized in that** said separation step C1) is carried out by means of a filter press.

3. The method as claimed in either one of the preceding claims, **characterized in that**, prior to said separation step C1), the method comprises a step during which the temperature of said fermented mixture (MF) is brought to a separation temperature (T) of between approximately 55°C and approximately 65°C.

4. The method as claimed in claim 1, **characterized in that** the pH of said fermented mixture (MF) is increased by adding at least one alkaline component (CAL).

5. The method as claimed in either of claims 1 and 2, **characterized in that** it comprises a substep C2) of washing said solid phase (PS) separated from said fermented mixture (MF).

6. The method as claimed in the preceding claim taken in combination with claim 2, **characterized in that** said step C2) of washing the solid phase (PS) separated from the fermented mixture (MF) is carried out by injection of washing water into the filter press in such a way that at least a part of the washing liquid (LL) having an ethanol content is automatically added to said liquid phase (PL) of the fermented mixture to be distilled.

7. The method as claimed in any one of the preceding claims, **characterized in that** it comprises a step E2) of producing at least a second fuel, which consists in dehumidifying said solid phase (PS) of said fermented mixture (MF) so as to produce a block (F2) of material of which the proportion by weight of solids is greater than 50%, and **in that** said block (F2) is capable of being burned, entirely or in part, in a boiler (CH), and/or **in that** said block (F2) is capable of being used, entirely or in part, for the production of a product (DDG) used in particular for animal feed.

8. The method as claimed in the preceding claim, **characterized in that** said solid phase (PS) of said fermented mixture (MF) is dehumidified by drying (H), and **in that** the gases (G) emitted during the drying are processed so as to extract, entirely or in part, the ethanol that these gases contain, in particular by washing the gases with water.

9. The method as claimed in claims 7 and 8, **characterized in that** said methane (F1), entirely or in part, and said second fuel (F2), entirely or in part, are burned in the same boiler.

**10.** The method as claimed in any one of the preceding claims, **characterized in that** said fermented mixture (MF) is obtained by means of the successive steps consisting in:

- A) preparing a paste comprising the plant starting material (MPV) capable of being fermented;
- B) bringing about the fermentation of said paste with a view to obtaining a fermented mixture (MF).


**Patentansprüche**

**1.** Verfahren zur Produktion von Bioethanol und zur Koproduktion von Energie aus einem stärkehaltigen, pflanzlichen Ausgangsmaterial (MPV),
**dadurch gekennzeichnet, dass** es mindestens die folgenden, aufeinanderfolgenden Schritte aufweist, die aus folgendem bestehen;

- A) - B)Gewinnen einer fermentierten Mischung aus dem pflanzlichen Ausgangsmaterial (MPV);
- C1) Trennen der flüssigen Phase (PL) und der festen Phase (PS) der fermentierten Mischung (MF) durch Filtration und Pressen, so dass der Gewichtsanteils des trockenen Materials der festen Phase (PS) zwischen ungefähr 40 % und ungefähr 45 % liegt;
- D) Destillieren mindestens eines Teils der flüssigen Phase (PL) der fermentierten Mischung (MF), um Ethanol und Schlempe (VL) zu erhalten;
- E1) Herstellen von Methangas (F1) - durch Verwendung der gesamten Schlempe (VL) - das einen ersten Brennstoff zur Koproduktion von Energie darstellt;

und dass das Verfahren vor dem Schritt zur Trennung C1) folgendes aufweist:

einen Schritt, im Verlaufe dessen der pH-Wert der fermentierten
Mischung (MF) auf einen Wert zwischen ungefähr 5,5 und 6,5 erhöht wird;
einen Schritt, im Verlaufe dessen der fermentierten Mischung (MF)

ein Filtrationszusatz (ADJ) hinzugefügt wird.

**2.** Verfahren nach Anspruch 1, **dadurch gekennzeichnet, dass** der Schritt der Trennung C1) mittels einer Filterpresse durchgeführt wird.

**3.** Verfahren nach einem der vorhergehenden Ansprüche, **dadurch gekennzeichnet, dass** das Verfahren vor dem Schritt zur Trennung C1) einen Schritt aufweist, im Verlaufe dessen die Temperatur der fermentierten Mischung (MF) auf eine Trenntemperatur (T) gebracht wird, die zwischen ungefähr 55 °C und ungefähr 65 °C liegt.

**4.** Verfahren nach Anspruch 1, **dadurch gekennzeichnet, dass** der pH-Wert der fermentierten Mischung (MF) durch Zusatz von mindestens einem alkalischen Bestandteil (CAL) erhöht wird.

**5.** Verfahren nach einem der Ansprüche 1 oder 2, **dadurch gekennzeichnet, dass** es getrennt von der fermentierten Mischung (MF) einen Unterschritt C2) des Spülens der festen Phase (PS) aufweist.

**6.** Verfahren nach dem vorhergehenden Anspruch zusammen mit Anspruch 2, **dadurch gekennzeichnet, dass** der Schritt C2) des Spülens der getrennten, festen Phase (PS) der fermentierten Mischung (MF) durch Einspritzen von Spülwasser in die Filterpresse durchgeführt wird, so dass mindestens ein Teil der Spülflüssigkeit (LL) mit Ethanolgehalt der flüssigen Phase (PL) der zu destillierenden, fermentierten Mischung automatisch zugefügt wird.

**7.** Verfahren nach einem der vorhergehenden Ansprüche, **dadurch gekennzeichnet, dass** es einen Schritt E2) der Produktion von mindestens einem zweiten Brennstoff aufweist, der im Entfeuchten der festen Phase (PS) der fermentierten Mischung (MF) besteht, um einen Materialblock (F2) herzustellen, dessen Gewichtsanteil an trockenem Material mehr als 50 % beträgt, und dass der Block (F2) ganz oder teilweise in einem Heizkessel (CH) verbrannt werden kann, und / oder dass der Block (F2) ganz oder teilweise für die Herstellung eines Produktes (DDG) verwendet werden kann, das insbesondere als Futtermittel genutzt wird.

**8.** Verfahren nach dem vorhergehenden Anspruch, **dadurch gekennzeichnet, dass** die feste Phase (PS) der fermentierten Mischung (MF) durch Trocknung (H) entfeuchtet wird, und dass die Gase (G), die während der Trocknung ausströmen, aufbereitet werden, um ganz oder teilweise Ethanol - das diese Gase enthalten - zu extrahieren, insbesondere durch Waschen der Gase mit Wasser.

9. Verfahren nach den Ansprüchen 7 und 8, **dadurch gekennzeichnet, dass** das Methan (F1) ganz oder teilweise und der zweite Brennstoff (F2) ganz oder teilweise in einem zweiten Heizkessel verbrannt werden.

10. Verfahren nach einem der vorhergehenden Ansprüche, **dadurch gekennzeichnet, dass** man die fermentierte Mischung (MF) durch aufeinanderfolgende Schritte erhält, die aus folgendem bestehen:

- A) Herstellen einer Paste aus einem pflanzlichen Ausgangsmaterial (MPV), das fermentiert werden kann;
- B) Herbeiführen der Fermentation der Paste, um die fermentierte Mischung (MF) zu erhalten.

**MPV**

**A)** Broyage

**A)** Liquéfaction
Saccharification

**B)**
Fermentation

**MF**

**CAL**
**ADJ**

Traitement du
mélange fermenté
**Ph   T**

**C1)**
Filtration
Pressage

**PS**

**C2)**
Lavage de
la PS

**BLOC**

**E2)**

Déshumidification
du BLOC  **H**

**F2**

**R**

**FUM**

Chaudière
**CH**
**PG**

**G**

Lavage des
gaz    **G**

**LL**

**Eau**

Recyclage des vinasses
(VL + Flegmasses)

**PL**

**D) Distillation**

**VL**

**Ethanol**

**F1 Méthane**

Rectification

**E1)**
Méthanisation

Flegmasses

Déshydratation de l'éthanol

**Boues**

Traitement
aérobie

**Bioéthanol**

Effluents

15

**EP 2 035 568 B1**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 4337123 A
- EP 0048061 A2
- WO 2004113549 A1